# EUROPEAN PATENT SPECIFICATION

(11) **EP 1 196 170 B1**
(45) Date of publication and mention of the grant of the patent: **14.07.2004**
(21) Application number: 00946165.8
(22) Date of filing: 19.07.2000
(51) Int. Cl.: A61K 31/436, A61P 25/28, A61P 25/00, A61P 9/10

(54) **USE OF A MACROLIDE COMPOUND FOR THE MANUFACTURE OF A MEDICAMENT FOR THE TREATMENT OF BRAIN DAMAGE CAUSED BY ISCHEMIA OR HEMORRHAGE**
VERWENDUNG EINER MAKROLID-VERBINDUNG ZUR HERSTELLUNG EINES MEDIKAMENTS ZUR BEHANDLUNG VON GEHIRNSCHÄDEN AUFGRUND VON ISCHÄMIE ODER BLUTUNGEN
UTILISATION D'UN COMPOSE DE MACROLIDE POUR L'OBTENTION D'UN MEDICAMENT POUR LE TRAITEMENT DES LESIONS CEREBRALES ISCHEMIQUES OU HEMORRAGIQUES

(30) Priority: 21.07.1999 GB 9917158
(43) Date of publication of application: 17.04.2002
(73) Proprietor: FUJISAWA PHARMACEUTICAL CO., LTD., Osaka-shi Osaka 541-8514 (JP)
(72) Inventor: JONES, Paul Alexander, Edinburgh EH8 9JZ (GB); SHARKEY, John, Edinburgh EH8 9JZ (GB); KELLY, John Shearer, Edinburgh EH8 9JZ (GB)
(74) Representative: Gaunt, Robert John
(86) International application number: PCT/GB2000/002788
(87) International publication number: WO 2001/005385

(56) References cited:
- WO-A-91/04025
- WO-A-93/14771
- WO-A-94/04148
- US-A- 5 648 351

## Description

### TECHNICAL FIELD

This invention relates to a new use of a macrolide compound.

### BACKGROUND ART

A certain macrolide compound, i.e., tacrolimus, and its related compounds are known to have preventing or treating activity of cerebral infarction (USP 5,648,351). However, it is desirable to provide more effective and/or safer drug with a superior pharmaceutical profile against cerebral ischemic disease.

### DISCLOSURE OF INVENTION

The inventors of this invention have found that one of the tacrolimus analogues, i.e., a compound (I), mentioned below, has an excellent neuroprotective efficacy. More specifically, it is effective for the prevention or treatment of brain damage caused by ischemia or hemorrhage

Accordingly, this invention provides a new use of the compound (I) for manufacturing an agent for the prevention or treatment of brain damage caused by ischemia or hemorrhage.

The tacrolimus analogue used in the present invention has the following chemical formula. 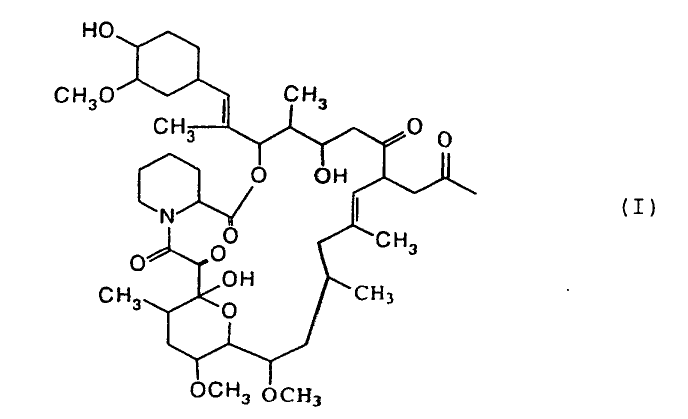 It has already been produced in USP 5,376,663,example 29.

With respect to the compound (I) used in the present invention, it is to be understood that there may be conformers and one or more stereoisomers such as optical and geometrical isomers due to asymmetric carbon atom(s) or double bond(s) , and such conformers and isomers are also included within the scope of the compound in the present invention. And further, the compound can be in the form of a solvate . The solvate preferably include a hydrate and an ethanolate.

The compound (I) usable in the present invention may be administered as pure compound or mixture of compound or preferably, in a pharmaceutical vehicle or carrier.

The compound (I) in this invention can be used in the form of a pharmaceutical preparation, for example, in solid, semisolid or liquid form, which contains the compound(I), as an active ingredient, in admixture with an organic or inorganic carrier or excipient suitable for external(topical), enteral, intravenous, intramuscular, or parenteral applications. The active ingredient may be compounded, for example, with the usual non-toxic, pharmaceutically acceptable, carriers for tablets, pellets, capsules, eye drops, suppositories, solutions (saline, for example), emulsion, suspensions (olive oil, for example), ointment, aerosol sprays, cream, skin plasters, patches and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an effective amount sufficient to produce the desired effect upon the process or condition of the disease.

Mammals to which the administration of the agent of the present invention may be suitable include livestock mammals such as cows, horses, etc., domestic animals such as dogs, cats, rats, etc. and humans.

For applying this composition to a human, it is preferable to apply it by injection.

While the dosage of therapeutically effective amount of the macrolide compounds varies from and also depends upon the age and condition of each individual patient to be treated, a daily dose of about 0.0001-1000 mg, preferably 0.001-500 mg and more preferably 0.01-100 mg. of the active ingredient is generally given for treating diseases, and an average single dose of about 0.001-0.01mg, 0.2-0.5 mg, 1 mg, 5 mg, 10 mg, 50 mg, 100 mg, 250 mg and 500 mg is generally administered. Daily doses for chronic administration in humans will be in the range of about 0.1-30 mg/kg/day.
And further, the compound (I) can be applied, simultaneously, separately or sequentially, with other agents having neuroprotective activity, such as thrombolytics (e.g., tPA, urokinase, etc), fibrinolytics, platelet inhibitors and so on.

The following examples illustrate the present invention in further detail.

### Example 1

Neuroprotective efficacy of the compound (I) in the rat endothelin-induced MCA occlusion model

### (1) METHOD

The compound (I) was dissolved in a polyoxyethylene-hydrogenated castor oil 60/ethanol (400mg/1ml) solution and administered at 1 and 3 mg.kg⁻¹. All drugs and relevant control were administered in a volume of 2 ml.kg⁻¹. MCA occlusion by the endothelin method was performed on male Sprague Dawley rats (271 - 324g) as described in USP 5,648,351. All drugs were infused through the i.v. catheter at 1 ml min⁻¹, five minutes post-lesion. The animals were sacrificed by cardiac infusion under barbiturate anaesthesia. Volume of lesion was calculated from measured areas of damage (as assessed three days post-lesion) using the Trapezoid Rule. Results are presented as volume (mm³) ± SEM. Statistical analysis was performed using ANOVA and post hoc Student-Newman-Keuls test, where p < 0.05 was set as an acceptable level for significance.

### (2) RESULT

Protection in the ET-1 model of stroke by the compound (I) at 1 mg.kg⁻¹ (n=14) and 3 mg.kg⁻¹ (n=9) against vehicle (n=11) was studied. The compound (I) protected the cortex 61% and 42% respectively at both 1 and 3 mg.kg⁻¹.

The compound (I) was proved to have a neuroprotective efficacy, though it has no immunosuppressive activity. So, the present invention provides a useful neuroprotective agent for preventing or treating brain damage caused by ischemia or hemorrhage.

So, it is useful when the following diseases or injury occur; that is, cerebral infarction, hemorrhage infarct, head injury, hemorrhage in brain such as subarachnoid hemorrhage or intracerebral hemorrhage, cerebral thrombosis, cerebral embolism, cardiac arrest, stroke (such as, acute, subacute, or chronic stroke) or transient ischemic attacks (TIA).

## Claims

1. A use of a compound of the following formula: 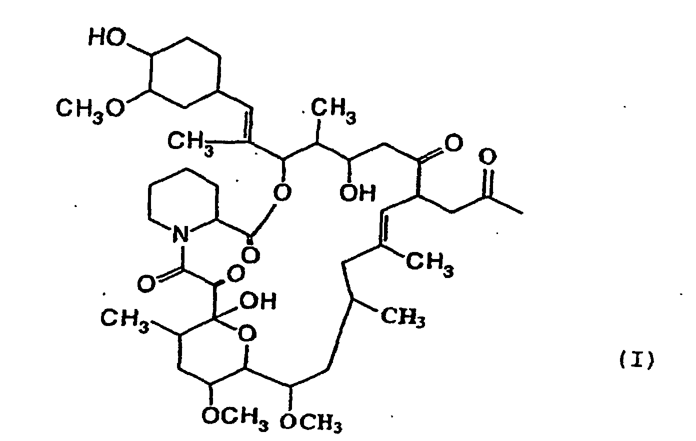 for manufacturing a medicament for the prevention or treatment of brain damage caused by ischemia or hemorrhage.

2. The use of claim 1, in which the brain damage caused by ischemia or hemorrhage is brain damage caused by cerebral infarction.

3. The use of claim 1, in which the brain damage caused by ischemia or hemorrhage is brain damage caused by hemorrhage infarct, head injury, subarachnoid hemorrhage, intracerebral hemorrhage, cerebral thrombosis, cerebral embolism, cardiac arrest, stroke, or transient ischemic attacks (TIA).

## Revendications

1. Utilisation d'un composé de la formule suivante : 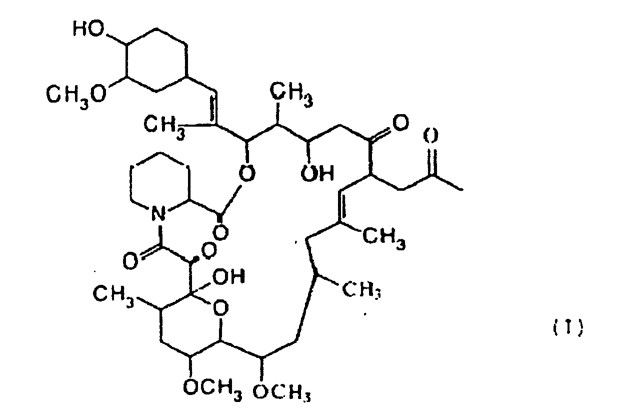 pour la fabrication d'un médicament pour la prévention ou le traitement d'un dommage cérébral provoqué par une ischémie ou une hémorragie.

2. Utilisation selon la revendication 1, dans laquelle le dommage cérébral provoqué par une ischémie ou une hémorragie est un dommage cérébral provoqué par un infarctus cérébral.

3. Utilisation selon la revendication 1, dans laquelle le dommage cérébral provoqué par une ischémie ou une hémorragie est un dommage cérébral provoqué par un infarctus hémorragique, un traumatisme crânien, une hémorragie sous-arachnoidienne, une hémorragie intracérébrale, une thrombose cérébrale, une embolie cérébrale, un arrêt cardiaque, un accident vasculaire cérébral, ou des accidents ischémiques transitoires (AIT).

## Patentansprüche

1. Verwendung einer Verbindung mit der folgenden Formel: 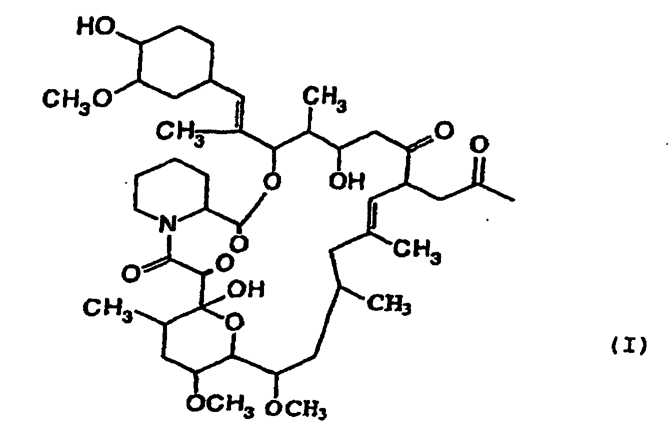 zur Herstellung eines Medikamentes zur Vorbeugung oder Behandlung von durch Ischämie oder Hämorrhagie verursachten Hirnschäden.

2. Verwendung nach Anspruch 1, wobei der durch Ischämie oder Hämorrhagie verursachte Hirnschaden ein durch Hirninfarkt verursachter Hirnschaden ist.

3. Verwendung nach Anspruch 1, wobei der durch Ischämie oder Hämorrhagie verursachte Hirnschaden ein Hirnschaden ist, der durch Hämorrhagieinfarkt, Kopfverletzungen, subarachnoide Hämorrhagie, Intracerebralhämorrhagie, Cerebralthrombose, Cerebralembolie, Herzstillstand, Schlaganfall oder transitorische ischämische Attacke (TIA) versachter Hirnschaden ist.
